# EUROPÄISCHE PATENTSCHRIFT

(11) **EP 1 976 979 B1**
(45) Veröffentlichungstag und Bekanntmachung des Hinweises auf die Patenterteilung: **10.08.2016**
(21) Anmeldenummer: 07722773.4
(22) Anmeldetag: 26.01.2007
(51) Int. Cl.: C12N 5/074, A61L 27/38

(54) **VERFAHREN ZUR HERSTELLUNG AUTONOM KONTRAHIERENDER HERZMUSKELZELLEN AUS ADULTEN STAMMZELLEN, INSBESONDERE HUMANEN ADULTEN STAMMZELLEN**
METHOD FOR PRODUCING AUTONOMOUSLY CONTRACTING CARDIAC MUSCLE CELLS FROM ADULT STEM CELLS, IN PARTICULAR HUMAN ADULT STEM CELLS
PROCÉDÉ POUR PRODUIRE DES CELLULES MYOCARDIQUES SE CONTRACTANT DE MANIÈRE AUTONOME À PARTIR DE CELLULES SOUCHES ADULTES, NOTAMMENT DE CELLULES SOUCHES ADULTES HUMAINES

(30) Priorität: 27.01.2006 DE 102006003996
(43) Veröffentlichungstag der Anmeldung: 08.10.2008
(73) Patentinhaber: Fraunhofer-Gesellschaft zur Förderung der angewandten Forschung e.V., 80686 München (DE)
(72) Erfinder: GULDNER, Norbert, W., 23564 Lübeck (DE); KRUSE, Charli, 23923 Herrnburg (DE); KAJAHN, Jennifer, 23552 Lübeck (DE)
(74) Vertreter: Katzameyer, Michael
(86) Internationale Anmeldenummer: PCT/EP2007/000694
(87) Internationale Veröffentlichungsnummer: WO 2007/085475

(56) Entgegenhaltungen:
- WO-A-02/057430
- WO-A-02/083864
- WO-A-2005/113747
- WO-A1-2005/001072
- MULLER-BORER BARBARA J ET AL: "Adult-derived liver stem cells acquire a cardiomyocyte structural and functional phenotype ex vivo" AMERICAN JOURNAL OF PATHOLOGY, Bd. 165, Nr. 1, Juli 2004 (2004-07), Seiten 135-145, XP009089522 ISSN: 0002-9440
- TOMITA S ET AL: "AUTOLOGOUS TRANSPLANTATION OF BONE MARROW CELLS IMPROVES DAMAGED HEART FUNCTION" CIRCULATION, AMERICAN HEART ASSOCIATION, DALLAS, TX, US, Bd. 110, Nr. SUPPL 19, 9. November 1999 (1999-11-09), Seiten II-247, XP000869909 ISSN: 0009-7322
- JIHYUN YOON ET AL: "Transdifferentiation of mesenchymal stem cells into cardiomyocytes by direct cell-to-cell contact with neonatal cardiomyocyte but not adult cardiomyocytes" ANNALS OF HEMATOLOGY, SPRINGER-VERLAG, BE, Bd. 84, Nr. 11, 1. November 2005 (2005-11-01), Seiten 715-721, XP019333828 ISSN: 1432-0584
- KRUSE C ET AL: "Pluripotency of adult stem cells derived from human and rat pancreas" APPLIED PHYSICS A: MATERIALS SCIENCE AND PROCESSING, SPRINGER VERLAG, BERLIN, DE, Bd. A79, Nr. 7, 2004, Seiten 1617-1624, XP002331149 ISSN: 0947-8396
- KAJAHN J ET AL: "Human cardiomyogenic cells generated from adult human pancreatic stem cells" CYTOTHERAPY, Bd. 8, Nr. Suppl. 2, 2006, Seiten 50-51, XP009089381 & 2ND INTERNATIONAL CONFERENCE ON STRATEGIES IN TISSUE ENGINEERING; WURZBURG, GERMANY; MAY 31 -JUNE 02, 2006 ISSN: 1465-3249

## Beschreibung

### Hintergrund

Herzversagen ist eine Haupttodesursache in Industrieländern und beruht auf der Unfähigkeit von ausgewachsenen Herzmuskelzellen (Kardiomyozyten) zur Teilung und Wiederherstellung von geschädigtem Herzmuskel. Nachdem die therapeutische Verwendung von embryonalen Kardiomyozyten in den meisten Ländern verboten ist, könnten adulte humane Stammzellen eine alternative Möglichkeit für die regenerative Medizin darstellen.

Adulte Stammzellen verschiedenen Ursprungs und Knochenmarkszellen wurden bereits intramyokardial injiziert, um in Kardiomyozyten überführt zu werden. Jedoch induzierte ein solcher Zell-Zell-Kontakt lediglich in Tierexperimenten mesenchymale Stammzellen zur Differenzierung in Kardiomyozyten (WO 02/083864 A2; Tomita et al. in *Circulation,* 9. November, 1999). Jihyun Yoon et al. berichteten in Ann. Hematol. (2005) 84:715-721), dass mesenchymale Stammzellen von Ratten bei direktem Zell-Zell-Kontakt mit neonatalen Kardiomyozyten, nicht jedoch mit adulten Kardiomyozyten, zu Kardiomyozyten transdifferenzieren können. Muller-Borer et al. (American Journal of Pathology, Bd. 165, Nr. 1 (2004) zeigten, dass adulte Leberstammzellen aus der Ratte in Kontakt mit neonatalen Herzzellen ebenfalls einen Kardiomyzyten-Phänotyp entwickeln. WO 02/057430 A2 offenbart die Isolierung von Stammzellen aus humanem intestinalen Epithel und ein Protokoll zur Differenzierung dieser Zellen zu Kardiomyozyten bei Zugabe von FGF-2. Die Anreicherung der Kardiomyozyten soll durch Einführung eines Selektionsmarkers unter der Kontrolle eines zellspezifischen Promotors erfolgen. WO 2005/001072 A1 offenbart die spontane Differenzierung von adulten Stammzellen aus exokrinem Drüsengewebe in verschiedene Zelltypen, einschließlich Herzzellen.

Vor diesem Hintergrund bestand eine Aufgabe der Erfindung in der Bereitstellung eines alternativen und verbesserten Verfahrens mit dem Kardiomyozyten, insbesondere humane Kardiomyozyten, einfach, d.h. ohne vorherige Transfektion der Stammzellen, effizient und in hoher Ausbeute aus adulten Stammzellen gewonnen werden können.

Diese Aufgabe wurde nun erfindungsgemäß gelöst durch ein Verfahren zur Herstellung von Herzmuskelzellen durch Differenzierung aus adulten Stammzellen, die aus exokrinem Drüsengewebe aus dem Pankreas oder einer Speicheldrüse oder Schweißdrüse isoliert wurden. Demgemäß betrifft die Erfindung das Verfahren zur Herstellung von Herzmuskelzellen gemäß den Ansprüchen 1-9 sowie den Stammzellenpatch gemäß den Ansprüchen 10-16.

### Beschreibung der Erfindung

Die Erfinder haben festgestellt, dass die aus exokrinem Drüsengewebe isolierten adulten Stammzellen pluripotent sind und sowohl das Potential zur spontanen Differenzierung zu Herzmuskelzellen aufweisen als auch in der Lage sind, sich unter geeigneten stimulierenden Bedingungen überwiegend oder fast ausschließlich zu Herzmuskelzellen zu entwickeln. Exokrines Drüsengewebe stellt somit eine sehr effektive Quelle für breit differenzierungsfähige Stammzellen dar, aus denen die gewünschten Herzmuskelzellen erfolgreich mit guten Ausbeuten massenhaft erhalten werden können.

Das erfindungsgemäß verwendete exokrine Drüsengewebe kann von einem erwachsenen Organismus, juvenilen Organismus oder nicht-humanen fötalen Organismus, vorzugsweise einem postnatalen Organismus, stammen. Der Begriff "adult", wie in der vorliegenden Anmeldung verwendet, bezieht sich somit auf das Entwicklungsstadium des Ausgangsgewebes und nicht auf dasjenige des Donororganismus, aus dem das Gewebe stammt. "Adulte" Stammzellen sind nicht-embryonale Stammzellen.

Das exokrine Drüsengewebe wird aus einer Speicheldrüse, Schweißdrüse oder dem Pankreas isoliert. In einer stark bevorzugten Ausführungsform handelt es sich dabei um acinäres Gewebe. Ganz besonders bevorzugt stammt das acinäre Gewebe aus dem Pankreas, der Ohrspeicheldrüse oder Unterkieferspeicheldrüse.

Ein Vorteil des erfindungsgemäßen Verfahrens besteht darin, dass die Stammzellen effektiv aus lebenden Donororganismen, z. B. aus humanen Speicheldrüsen oder durch einen minimalinvasiven retroperitonealen Eingriff aus dem Pankreas, gewonnen werden können, ohne dass der Donororganismus entscheidend beeinträchtigt wird. Dies ist sowohl unter ethischen Gesichtspunkten als auch mit Blick auf die Möglichkeit der weiteren Beobachtung des Donororganismus hinsichtlich eventueller Krankheiten von besonderem Vorteil.

Gemäß einer ersten Ausführungsform der Erfindung werden die primär aus dem Organismus isolierten Stammzellen als Quelle für die weitere Kultivierung und Differenzierung hin zu Herzmuskelzellen verwendet. Diese Variante besitzt den Vorteil einer besonders einfachen Verfahrensführung. Die gewünschten differenzierten Zellen können direkt aus einer Primärkultur gewonnen werden. Alternativ ist gemäß einer abgewandelten Ausführungsform der Erfindung vorgesehen, dass zunächst eine Aggregation der aus dem Organismus isolierten Stammzellen zu so genannten organoiden Körpern erfolgt. Diese Variante besitzt den Vorteil, dass mit den organoiden Körpern ein effektives Reservoir für größere Mengen differenzierter Zellen geschaffen wird. Die Erfinder haben festgestellt, dass die aus dem exokrinen Drüsengewebe isolierten Stammzellen organoide Körper bilden, die bei Nährstoffversorgung ein starkes Wachstum zu Gewebekörpern mit Durchmessern bis zu einigen Millimetern oder darüber zeigen.

Grundsätzlich kann das erfindungsgemäße Verfahren ausgeführt werden, indem Herzmuskelzellen, die sich spontan aus den primär oder sekundär (aus den organoiden Körpern) isolierten Stammzellen gebildet haben, identifiziert, erforderlichenfalls selektiert und weiter vermehrt werden. Gemäß der Erfindung ist bei der Differenzierung der Herzmuskelzellen eine Stimulation der Zellkultur vorgesehen. Die Stimulation besitzt den Vorteil einer erhöhten Effektivität und Geschwindigkeit der Bildung der gewünschten Herzmuskelzellen. Gemäß einer ersten Variante ist nach der Differenzierung der Stammzellen zu den Herzmuskelzellen deren stimulierte Vermehrung in einem Kultivierungsmedium vorgesehen. Gemäß einer zweiten Variante geschieht die Stimulation bereits in einem früheren Stadium und betrifft die noch undifferenzierten Stammzellen, deren Entwicklung/Differenzierung zu den gewünschten Herzmuskelzellen veranlasst wird.

Erfindungsgemäß kann die Stimulation eine oder mehrere der folgenden Stimulationsbehandlungen umfassen, die gleichzeitig oder aufeinander folgend durchgeführt werden können. Es kann eine Co-Kultivierung mit differenzierten Herzmuskelzellen oder mit davon abgeleiteten Zelllinien, eine Behandlung (Prägung) mit immobilisierten oder gelösten, in der flüssigen Phase bereitgestellten molekularen Differenzierungsfaktoren oder eine Genaktivierung in der Stammzelle vorgesehen sein. Des weiteren kann eine Stimulation den Zusatz von anderen Substanzen, zum Beispiel Hormone (z.B. Insulin), oder Zelltypen, welche die Differenzierung beeinflussen, beinhalten.

Wenn die Prägung mit immobilisierten Wachstumsfaktoren erfolgt, so werden vorzugsweise Differenzierungsfaktoren verwendet, die auf einem beweglichen, relativ zu den Stammzellen positionierbaren Träger fixiert sind. Vorteilhafterweise kann damit eine gezielte Differenzierung einzelner Stammzellen oder bestimmter Stammzellgruppen erreicht werden. Der Träger ist beispielsweise ein synthetisches Substrat, das Vorteile für eine gezielte Gestaltung mit den Differenzierungsfaktoren besitzt, oder eine biologische Zelle, auf deren Zellmembran die Differenzierungsfaktoren angeordnet sind.

Einige beispielhafte, nicht-beschränkende Wachstums- bzw. Differenzierungsfaktoren, die verwendet werden können, sind 5'-Azazytidin, bFGF, Cardiogenol, Transferrin und PDGF.

In einer speziellen Ausführungsform der Erfindung geschieht die Stimulationsbehandlung durch Kultivierung der Stammzellen unter normalen Bedingungen (z.B. wie in Beispiel 1 beschrieben) in Gegenwart von biologischen "nanostrukturierten Oberflächen". Dieser Begriff bezeichnet hier Zellen, z.B. Kardiomyozyten oder andere Herzzellen, die durch eine Fixierungsbehandlung, z.B. mit Formaldehyd oder einem anderen geeigneten Fixierungsmittel, abgetötet wurden und deren Zellmembran dabei undurchlässig gemacht wurde, während die Oberflächenstruktur der Zellen, einschließlich der Oberflächenproteine und anderer dort exponierter Moleküle, erhalten blieb. Auf diese Weise ist ein Einfluss von Substanzen aus dem Inneren dieser Zellen ausgeschlossen und die Stimulation erfolgt spezifisch durch den Einfluss der Oberflächenstruktur der fixierten Zellen.

Wenn gemäß einer weiteren bevorzugten Ausführungsform der Erfindung eine Identifizierung und Selektion der differenzierten Zellen aus der Zellkultur vorgesehen ist, können sich Vorteile für die weitere Verwendung der gebildeten Herzmuskelzellen ergeben. Es kann insbesondere eine Zellzusammensetzung bereitgestellt werden, die vollständig oder zum größten Teil aus Herzmuskelzellen besteht. Wenn die Selektion mit Sortierverfahren erfolgt, die an sich bekannt sind, wie z. B. mit einem präparativen Zellsorter-Verfahren oder eine Sortierung in einem fluidischen Mikrosystem, können sich Vorteile für die Kompatibilität mit herkömmlichen zellbiologischen Prozeduren ergeben.

Ein weiterer Vorteil der Identifizierung und Selektion besteht darin, dass Zellen, die nicht als Herzmuskelzellen identifiziert sind und entsprechend nicht aus der bearbeiteten Kultur selektiert werden, einer weiteren Kultivierung und Differenzierung unterzogen werden können. Damit kann vorteilhafterweise die Ausbeute des erfindungsgemäßen Verfahrens gesteigert werden.

Möglichkeiten zur Sortierung von Kardiomyzyten und ihren Vorläuferzellen wären z.B. mittels Transfektion von Reportergenkonstrukten mit herzspezifischen Promotoren, welche zu fluoreszierenden Produkten führen, wenn sie eingeschaltet werden, oder fluoreszenzmarkierte Antikörper gegen herzspezifische Proteine.

Gemäß der Erfindung werden zur Bildung der Herzmuskelzellen Stammzellen aus Gewebe von Speicheldrüse, Schweißdrüse oder Pankreas des Organismus gewonnen. Die Stammzellen werden im Falle von Pankreas oder Speicheldrüse insbesondere aus Gewebe isoliert, das aus acinärem Gewebe besteht oder acinäres Gewebe enthält. Bei der Gewinnung aus der Pankreas können sich Vorteile für die Verwendung weiterer Gewebeteile des Pankreas für die genannte Stimulation ergeben. Bei der Gewinnung aus der Speicheldrüse können sich Vorteile für die schonende Behandlung des Donororganismus ergeben.

Bevorzugte Donororganismen sind Wirbeltiere, insbesondere Säuger. Ganz besonders bevorzugt ist der Mensch. Bei der Verwendung humaner Stammzellen erfolgt die Isolation der Stammzellen aus nicht-embryonalen Zuständen, also aus differenziertem Gewebe in der juvenilen Phase oder der adulten Phase. Bei nicht-menschlichen Donororganismen kann grundsätzlich auch auf differenziertes Gewebe im fötalen Zustand zurückgegriffen werden.

Die erfindungsgemäß hergestellten Herzmuskelzellen werden vorzugsweise therapeutisch verwendet. Ein besonderer Vorteil der vorliegenden Erfindung liegt darin, dass erstmals humane Herzmuskelzellen aus nicht-embryonalen Stammzellen hergestellt und für die Behandlung von Menschen eingesetzt werden können. Eine besonders attraktive Möglichkeit ist die autologe Behandlung eines Menschen mit Herzmuskelzellen, die von eigenen Stammzellen gewonnen wurden. Auf diese Weise können wirksam Abstoßungsreaktionen vermieden werden. Typischerweise wird die Behandlung die Wiederherstellung von verletztem oder geschädigtem Myokard beinhalten. Die Behandlung kann entweder die Verabreichung der undifferenzierten Stammzellen und deren induzierte Differenzierung zu Herzmuskelzellen im Körper beinhalten oder die Verabreichung der bereits differenzierten Herzmuskelzellen, z.B. in einem Transplantat.

Mit dem erfindungsgemäßen Verfahren erhältlich sind sowohl isolierte Herzmuskelzellen, die aus einer Stammzelle differenziert wurden, welche aus differenziertem exokrinen Drüsengewebe eines Organismus stammt, als auch eine Zellzusammensetzung, die eine Vielzahl derartiger Herzmuskelzellen enthält. Die Zellzusammensetzung kann weitere Zellen oder Materialien enthalten, die z.B. eine Matrix bilden. Die Zellzusammensetzung kann auch eine Umhüllung oder eine 3-dimensionale Matrix umfassen, in der die Herzmuskelzellen und gegebenenfalls weitere Zelltypen angeordnet sind. Die Umhüllung oder 3-dimensionale Matrix besteht zum Beispiel aus Alginat, Kollagen, implantierbaren Materialien, Polymeren (Biopolymere oder synthetische Polymere), insbesondere im Körper abbaubaren Materialien.

Gemäß einer besonders bevorzugten Ausführungsform der Erfindung handelt es sich bei den verwendeten adulten Stammzellen um humane Stammzellen, die aus Pankreasgewebe isoliert wurden.

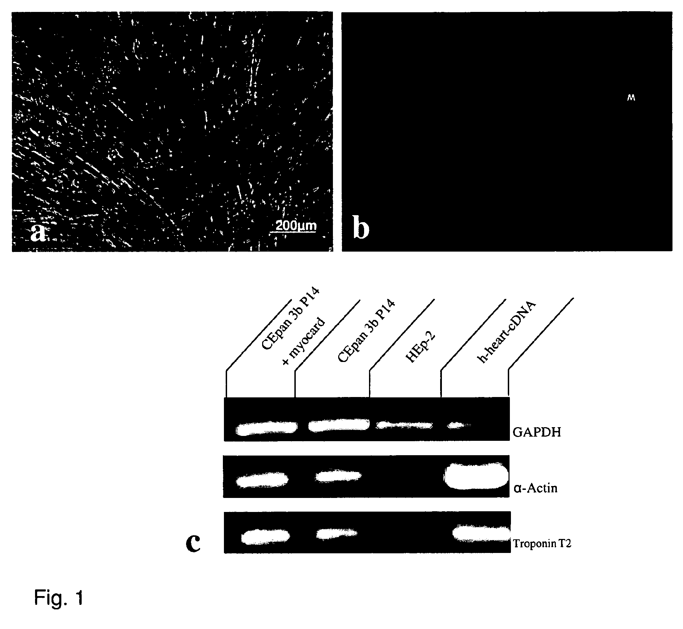

Adulte Stammzellen wurden aus pankreatischem Gewebe von Patienten, die sich einer Pankreasoperation unterzogen hatten, isoliert und kultiviert (hinsichtlich der Bedingungen siehe Beispiel 1). Die Zellen wurden selektiert, mit Medium (z.B. DMEM) mit fetalem Kalbserum kultiviert und bis zu mehr als 25 mal passagiert. Die Kulturen konnten zwischen einzelnen Passagen auch eingefroren werden, ohne die Zellen zu beinträchtigen. Die Kulturen zeigten in verschiedenen Passagen spontan gebildete netzartige Zellcluster (Fig. 1a) und einige dieser Zellcluster zeigten zelluläre Kontraktionen an verschiedenen Stellen, was ein funktionelles kontraktiles System anzeigt.

In einem optimierten Verfahren, mit dem schneller größere Mengen an kontrahierenden Herzmuskelzellen (Kardiomyozyten) erhalten werden konnten, wurden pankreatische Stammzellen mit kleinen Stücken von humanem Herzmuskel, erhalten bei einer Herzklappenoperation, kokultiviert. Nach einer Kontaktzeit von 48 Stunden wurde das Myokard entfernt und Stammzellen wurden für weitere 2 bis 4 Tage bzw. 2 Wochen in Kultur gehalten, um den Einfluss des Myokards auf die Differenzierung zu Kardiomyozyten zu untersuchen. Danach wurden die verschiedene Verfahren, einschließlich Immunzytochemie von Sarkomeren und herz-spezifischem Troponin I, semiquantitativer RT-PCR-Analyse hinsichtlich Alpha-Actin und Troponin T2 und elektronenmikroskopische Untersuchung angewandt, um Kardiomyozyten nachzuweisen.

Myokard für die Kokultivierung kann mittels Biopsien aus dem Herz-Septum, die bereits routinemäßig zum Nachweis von Gewebeabstoßung nach einer Herztransplantation verwendet werden, erhalten werden. Das erfindungsgemäße Verfahren, mit dem eine große Anzahl von kontraktionsfähigen Kardiomyozyten auf einfache und bequeme Weise hergestellt werden kann, könnte für die allgemeine Myokardregeneration von Bedeutung sein und insbesondere für kontraktionsfähige Myokard-Patches.

### Figurenbeschreibung

Die Figuren 1 und 2 zeigen die Ergebnisse verschiedener Nachweisverfahren für Kardiomyozyten.
**Fig. 1a** Kulturen von pankreatischen Stammzellen mit netzartigen Zellclustern zeigen autonome Kontraktionen.
**Fig. 1b** Immunzytochemische Visualisierung von Sarkomeren (rot) in transformierten adulten pankreatischen Stammzellen (blaue Kerne) in Kontakt mit humanem Myokard (M) für 2 Tage. Ein abnehmender Gradient von M bis zur Peripherie ist zu beobachten.
**Fig. 1c** Eine Genexpressionsanalyse mit den herzspezifischen PCR-Primern für die Zielgene α-Aktin und Troponin T2-Isoform- 1 demonstriert eine stärkere Zunahme von muskelzell-spezifischen Molekülen in kokultivierten Zellen (CEpan 3b, humane pankreatische Stammzellen; P14, Passage 14; HEp-2, humane karzinogene Zelllinie; h-heart-cDNA, humane Herz-cDNA).
**Fig. 2a,b** Humane pankreatische adulte Stammzellen mit immunzytochemischer Anfärbung für herzspezifisches Troponin I ohne Kontakt mit humanem Myokard (a) und nach einem zweitägigen Kontakt mit humanem Myokard (b). Ein klarer Beleg der Existenz von herzspezifischem Troponin I in transformierten Zellen wird demonstriert.
**Fig. 2c,d** Verschiedene Stadien von Kardiomyozyten, transformiert aus adulten pankreatischen Stammzellen, sind in den elektronenmikroskopischen Aufnahmen vier Tage nach einem 48 stündigen Kontakt mit Biopsien von humanem Myokard gezeigt. Myofilamente und Strukturen von teilweiser (c) und vollständiger (d) Entwicklung der Glanzstreifen sind gezeigt. Vesikel, organisiert in Linien (Fig. 2c, Pfeile), werden als Querschnitte eines prämaturen Status des sarkoplasmatischen Retikulums betrachtet.
**Fig. 3** zeigt die Platzierung eines biodirektional transformierbaren Stammzellenpatches (BTS) zwischen Myokard und dem breiten Rückenmuskel (Musculus latissimus dorsi) für die Myokardregeneration.

Eine spezielle Anwendungsmöglichkeit der vorliegenden Erfindung betrifft einen bidirektional transformierbaren Stammzellenpatch (BTS) für die Myokardregeneration. Ein solcher Patch umfasst adulte Stammzellen aus exokrinem Drüsengewebe, vorzugsweise pankreatische Stammzellen, und eine poröse, gegegebenenfalls unterteilte, Matrix zur Aufnahme der Zellen, besitzt eine grosse Auflagefläche für die Myokardwundfläche, auf die es nach Entfernung des Epikards aufgebracht werden soll, ist in der Regel mehrlagig, z.B. aus mehreren schwammartigen Membranen aufgebaut, aber relativ dünn (geringe Diffusionsstrecke) und gut fixierbar.

Die poröse Matrix ist z.B. eine Kollagenmatrix oder besteht aus einem anderen physiologisch annehmbaren Material. In einer Ausführungsform sind alle Materialien des Patches im Körper abbaubar.

Der Patch kann ferner ganz oder teilweise zu Herzmuskelzellen ausdifferenzierte Zellen oder andere im Herzen vorkommende differenzierte Zellen enthalten. Der Patch kann ferner Substanzen enthalten, welche die Differenzierung der Stammzellen zu Kardiomyozyten fördern, und/oder pharmazeutische Wirkstoffe, z.B. zur Unterdrückung einer Abstoßungsreaktion.

Der Begriff "bidirektional transformierbar", wie hier verwendet, bedeutet, dass der Patch so gestaltet ist, dass die in diesem Patch enthaltenen Zellen, insbesondere Stammzellen, auf beiden Seiten mit Zellen des angrenzenden Gewebes oder von den Zellen abgegebenen Substanzen in Kontakt kommen können und dadurch eine Transformation/Differenzierung der Stammzellen zu dem gewünschten Zelltyp induziert oder stimuliert werden kann.

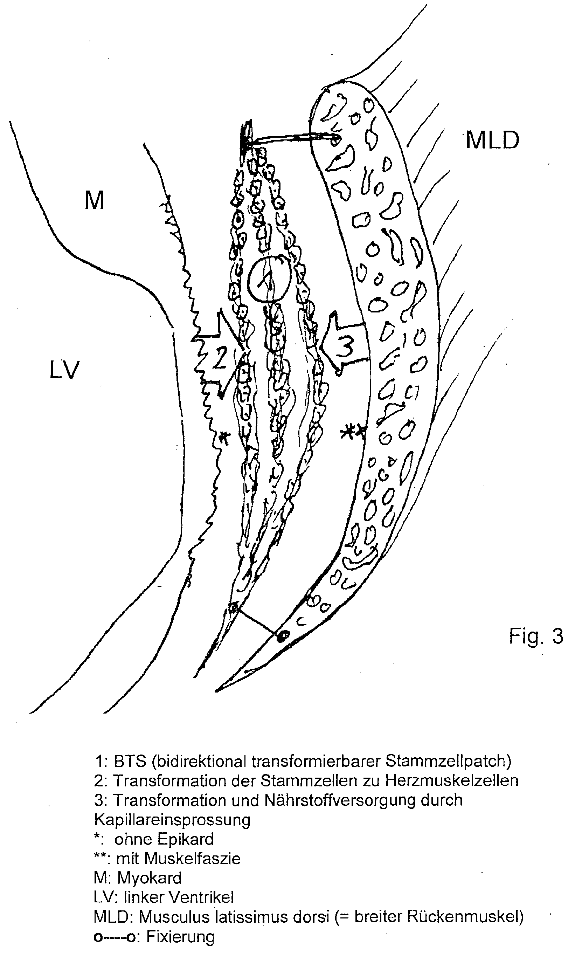

In einer bevorzugten Ausführungsform wird der Patch zwischen dem breiten Rückenmuskel (Musculus latissimus dorsi) und dem von Epikard befreiten Myokard platziert (siehe Fig. 3). Dann können die Zellen des Myokards bzw. davon abgegebene Substanzen eine Differenzierung der herzseitig im Patch angeordneten Stammzellen zu Herzzellen, insbesondere Herzmuskelzellen, induzieren. Auf der anderen Seite kann das Gewebe des Rückenmuskels die Zellen des Patches einerseits mit Nährstoffen versehen und andererseits eine Transformation der rückenseitig angeordneten Stammzellen zu Gefäßzellen, z.B. Endothelzellen etc., induzieren oder eine Einwanderung entsprechender Zellen in den Patch erlauben, so dass eine Neubildung von Kapillargefäßen im Patch oder dem angrenzenden Gewebe stattfinden kann. Gewünschtenfalls wird bei dem Patienten zusätzlich eine Hyperkapillarisierung des Rückenmuskelmantels mit intakter Muskelfaszie durch intermittierende transkutane Elektrostimulation (z.B. durch aufgeklebte Stimulationselektroden) induziert.

### BEISPIEL 1

### Isolierung, Kultivierung und Kokultivierung adulter pankreatischer humaner Stammzellen

Die Quelle des humanen pankreatischen Gewebes war gesundes Gewebe, das bei Pankreasoperationen wegen Krebs oder Entzündungserkrankungen aus Sicherheitsgründen entfernt worden war. Das Gewebe wurde in physiologischer Salzlösung erhalten. Daraus wurden Pankreas-Acini wie bereits beschrieben (DE 10328280; Orlic et al., Nature 410:701-705) isoliert.

Speziell wurde das pankreatische Gewebe mit Verdauungsmedium, enthaltend HEPES-Eagle-Medium (pH 7,4), 0,1 mM HEPES-Puffer (pH 7,6), 70 % (Vol./Vol.) modifiziertes Eagle-Medium, 0,5 % (Vol./Vol.) Trasylol (Bayer AG, Leverkusen, Deutschland), 1 % (Gew./Vol.) Rinderserumalbumin, 2,4 mM CaCl₂ und Collagenase (0,63 PZ/mg, Serva, Heidelberg, Deutschland) behandelt. Nach dem Verdau wurden die Acini durch Aufsaugen und Ausstoßen durch verschiedene Glaspipetten mit engen Öffnungen dissoziiert und durch ein Nylonsieb filtriert. Die Acini wurden zentrifugiert und weiter gereinigt durch Waschen in Dulbecco's modifiziertem Eagle's Medium (DMEM, Gibco, Deutschland), ergänzt mit 20 % fetalem Kalbserum (FCS), äquilibriert mit Carbogen und auf pH 7,4 eingestellt. Die Waschprozedur (Zentrifugation, Absaugen, Resuspension) wurde 5 mal wiederholt. Die Acini wurden in DMEM resuspendiert und bei 37° C in einer feuchten Atmosphäre mit 5 % CO₂ kultiviert. Nach 1 - 2 Tagen Kultur wurden spindelförmige Zellen beobachtet, welche die äußeren Ränder der Pankreas-Acini umgaben. Differenzierte acinäre Zellen wurden bei jedem Mediumaustausch entfernt. Nach dem Erreichen von Konfluenz wurden pankreatische Stammzellen durch Trypsinbehandlung kultiviert, gezählt und mit einer Dichte von 2,4 x 10⁵ Zellen/cm² erneut ausgesät. Diese Prozedur wurde wiederholt, bis ausreichend Zellen zur Verfügung standen. Wie bereits gezeigt werden konnte, finden keine Veränderungen der Stammzellen während der Passagen statt (durch Anfärbung überprüft). Deshalb verwendeten wir die Passagen 14 und 4 für die weitere Differenzierung.

Die Stimulierung der Differenzierung in Kardiomyozyten wurde erzielt durch Kokultivierung der primären Zellen mit jeweils 5 Stücken Myokard (4 x 4 x 4 mm) für 2 Tage. Das Gewebe (mitraler Papillarmuskel oder Aurikulum) wurde bei einer Operation zum Herzklappenersatz erhalten und in physiologischer Salzlösung transportiert. Die Herzmuskelstücke wurden am Boden der Kulturgefäße für 3 Stunden platziert, bis die Primärzellen (1 x 10⁶) aufgebracht wurden. Nach 48 Stunden wurden die Herzmuskelstücke entfernt und die Stammzellen weiter wie oben beschrieben kultiviert. Die Zellen wurden dann jedes Mal nach Erreichen von Konfluenz einer Passage unterworfen. Immunzytochemische Analysen wurden direkt 48 Stunden nach der Behandlung durchgeführt. Zur Untersuchung der Langzeitwirkungn der Differenzierung wurden die Zellen 17 Tage nach der Behandlung für PCR-Analysen gewonnen.

Als die Zellen in den Kulturschalen konfluent wurden, konnten netzartige Cluster beobachtet werden (Fig. 1a). Die Zellschicht wurde mit der weniger nährstoffreichen phosphatgepufferten Salzlösung (PBS) gewaschen und mechanisch teilweise vom Boden der Kultur mit einem Schaber abgehoben. Kontrahierende Regionen wurden dann auf einem Video dokumentiert.

Zur Überprüfung, ob Kardiomyozyten aus Biopsien von Herzgeweben auswachsen, wurden die Biopsien wie oben beschrieben, jedoch ohne pankreatische Stammzellen, kultiviert. Nach 2 Tagen konnten keine auswachsenden Zellen nachgewiesen werden.

### BEISPIEL 2

### Nachweis von Herzmuskelzellen

### 1. Immunzytochemie von Sarkomeren

Sowohl die stimulierten als auch die nichtstimulierten Stammzellen wurden auf Kammer-Objektträgern ausgesät und mindestens 2 Tage lang kultiviert, bevor sie mit Methanol:Aceton (7:3), enthaltend 1 g/ml DAPI (Roche, Schweiz), fixiert und 3 x in PBS gewaschen wurden. Nach Inkubation in 10 %igem normalen Ziegenserum bei Raumtemperatur für 15 Minuten wurden die Proben mit dem primären Antikörper über Nacht bei 4° C in einer Feuchtigkeitskammer inkubiert. Primärer monoklonaler Antikörper wurde gegen das Sarkomer-Myosin MF 20 (DSHB, USA) gerichtet. Nach dreimaligem Spülen mit PBS wurden die Träger 45 Minuten lang bei 37° C mit Cy3-markiertem Antimaus-IgG, 1:200 verdünnt, inkubiert. Die Objektträger wurden 3 x in PBS gewaschen, mit Vectashield-Montagemedium (Vector, USA) bedeckt und mit einem Fluoreszenzmikroskop (Axioskop Zeiss, Deutschland) analysiert. Um auszuschließen, dass nachgewiesene Sarkomere aus der Biopsie freigesetzt worden waren und an den Stammzellen hafteten, wurden Kontrollen mit Myofibroblasten und Endothelzellen mit Myokard kokultiviert. Bei diesen Kontrollen waren die getesteten Zellen gemäß Immunzytochemie negativ bezüglich Sarkomeren.

Dagegen wurde ein immunzytochemischer Nachweis von Sarkomeren erfolgreich bei transformierten adulten humanen pankreatischen Stammzellen in vier Ansätzen nach Kontakt mit humanem Myokard (M) von vier verschiedenen Patienten durchgeführt. Ein abnehmender Gradient von entwickelten Sarkomeren von "M" (Platzierung des Myokards) bis zur Peripherie wurde nach zwei Tagen Myokardial-Kontakt gezeigt(Fig. 1b).

### 2. Immunzytochemie von herz-spezifischem Troponin I

Stammzellen wurden mit Myokard-Biopsien für 48 Stunden kokultiviert und weitere 2 bis 4 Tage nach Entfernung des Myokards gezüchtet. Danach wurden die Proben 2 mal mit PBS gespült und 24 Stunden an Luft bei Raumtemperatur getrocknet und dann durch reines Aceton für 10 Minuten bei -20° C fixiert, erneut 2 x 5 Minuten mit TBS-Puffer gespült und mit RPMI 1640 mit 10 %igem AB-Serium vorinkubiert. Monoklonale Antitroponin I-Antikörper (Cone 2d5, Biozal 1:25) wurden als primäre Antikörper für 60 Minuten lang eingesetzt. Eine Verabreichung von sekundärem Antikörper (Antimaus-Kaninchen-Antikörper; DAKO; 1:25, für 30 Minuten) gefolgt von Inkubation mit einem Komplex mit alkalischer Phosphatase bzw. ohne alkalische Phosphatase (DAKO; 1:50, 30 Minuten) wurde mehrmals wiederholt. Schließlich wurde eine Substrat-Inkubation (Naphthol/- Neofuchsin) und Kontrastfärbung mit Hämalaun vor einer mikroskopischen Untersuchung vorgenommen. Zusätzlich erfolgte eine Isotopenkontrolle mit Maus-IgG 1 (DAKO) und für eine weitere negative Kontrolle wurde Skelettmuskel angefärbt. Myokard wurde als positive Kontrolle verwendet. Eine Isotypen-Kontrolle mit Maus IgG 1 (DAKO) war ebenfalls negativ. Zusätzliche Kontrollen, die mit Skelettmuskeln durchgeführt wurden, waren ebenfalls negativ. Wie erwartet, zeigte eine Kontrolle mit humanem Myokard ein positives Ergebnis (Daten nicht gezeigt).

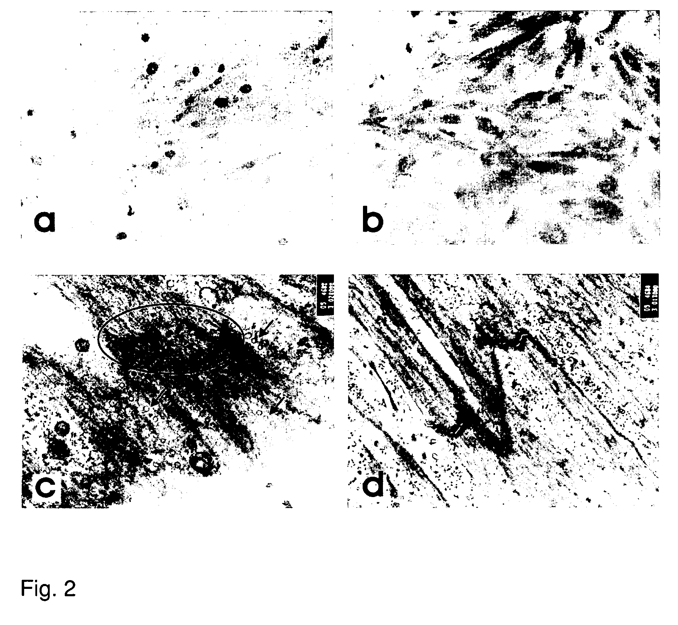

Die Immunzytochemie von Herz-spezifischem Troponin I war bereits 2 Tage nach einer 48-stündigen Kokultur mit einer humanen myokardialen Biopsie stark positiv, wie in Fig. 2b gezeigt. Stammzellen, die nicht in Kontakt mit myokardialen Biopsien waren, ergaben bei einem immunzytochemischen Nachweis für Troponin I hauptsächlich negative Ergebnisse und dienten als weitere Kontrolle (Fig. 2a).

### 3. Semiquantitative RT-PCR-Analyse

Gesamt-Zell-RNA wurde unter Verwendung eines Nucleo-Spin^{®} RNA II-Kits (Macherey-Nagel, Düren, Deutschland) isoliert. 0,5 µg Gesamt-RNA wurden unter Verwendung von reverser Transkriptase Superscript II RNase H⁻ (RT, Invitrogen) und oligo dT-Primern (Invitrogen) nach den Instruktionen des Herstellers revers in cDNA transkribiert. Die PCR-Reaktionen wurden in 50 µl Reaktionsvolumen unter Verwendung von Taq-DNA-Polymerase (MBI Fermentas) durchgeführt. Die Reaktionen wurden für 38 Zyklen durchgeführt. Ein Kontrolllauf von RNA ohne reverse Transkription erfolgte, um eine Kontaminierung mit genomischer DNA zu prüfen und ergab keine Banden. Zur Normalisierung der cDNA-Konzentration in verschiedenen RT-Proben maßen wir die relative Expression von GAPDH als repräsentative Kontrolle für ein internes Haushaltungsgen. Die erwarteten Fragmentgrößen und die optimalen PCR-Verschmelzungstemperaturen waren wie folgt: GAPDH, 5':gagtcaacggatttggtcgt, 3':ggaagatggtgatgggattt (213 bp, 58,8°C), Troponin T2, 5':gattctggctgagaggagga, 3':tggagactttctggttatcgttg (197 bp, 62,6°C), Alpha-Aktin, 5':gtgtgacgacgaggagacca, 3':cttctgacccatacccacca (154 bp, 62,6°C). Gereinigte humane Herz-RNA (Ambion) und eine karzinogene Zelllinie (HEp2) dienten als funktionelle Kontrollen für den PCR-Primer.

Eine semiquantitative RT-PCR-Analyse (Fig. 1c) für α-Aktin und Troponin T2 zeigte zwei Wochen nach Kontakt eine stärker ausgeprägte Erhöhung dieser muskelzell-spezifischen Moleküle als bei unbehandelten spontan differenzierten Stammzellen. Die Erhöhung von α-Aktin und Troponin T2 nach zwei Wochen war reproduzierbar und signifikant.

### 4. Elektronenmikroskopische Untersuchung

Zellen, die auf Deckgläsern gezüchtet worden waren, wurden mit 2,5 % Glutaraldehyd in 0,1 M Cacodylat-Puffer 1 h lang fixiert. Eine Nachfixierung wurde durchgeführt mit 1 % OsO₄ in 0,1 M Cacodylat-Puffer für 2 h; Proben wurden mit Ethanol entwässert und in Araldit (Fluka, Buchs, Schweiz) eingebettet. Ultradünne Schnitte wurden mit Uranylacetat und Bleicitrat (Ultrostainer Carlsberg System, LKB, Bromma, Schweden) angefärbt und mit einem Philips Elektronenmikroskop EM 400 bei 60 kV (Philips, Eindhoven, Niederlande) untersucht.

Die elektronenmikroskopische Untersuchung (Fig. 2cd) zeigt nach 48 Stunden Kontakt von adulten pankreatischen Stammzellen mit humanem Myokard und weiteren 4 Tagen Differenzierung Zellen mit einer Anzahl kontraktiler Fibrillen. Ferner waren verschiedene Stadien von Glanzstreifen zu beobachten. Während der Glanzstreifen in Fig. 2c nur schwach aber klar erkennbar ist, ist in Fig. 2d der Glanzstreifen gut differenziert wie in reifem Gewebe. Nachdem ein Glanzstreifen nur in Herzmuskel gefunden wird, belegen diese Befunde ebenfalls eine Differenzierung von adulten humanen Stammzellen in Kardiomyozyten.

Nach 14 - 40 Tagen Wachstum in Kultur und nach 48 Stunden in Kontakt mit humanem Myokard wurden die Zellen partial mechanisch von dem Kulturgefäß abgehoben und mit einem weniger nährstoffreichen Kulturmedium behandelt. Die Zellkomplexe zeigten Kontraktionen in verschiedenen Bereichen. Diese Kontraktionen waren autonom und in mehreren Kulturen reproduzierbar, was ein funktionales kontraktiles System demonstriert. Dies ist die erste Beobachtung von humanen autonom kontrahierenden Myokardzellen, welche aus humanen adulten Stammzellen erzeugt wurden.

### BEISPIEL 3

### Differenzierung mit 5-Azazytidin

Die Stammzellen werden in einer Dichte von 1 x 10³ in Petrischalen ausgesät und 24 h lang in DMEM (mit 10 % FKS und 1 % Penicillin/Streptomycin) kultiviert, bis sie adhärent am Boden der Kulturschalen haften. Daraufhin werden die Zellen 24 h lang in einem Differenzierungsmedium kultiviert, welches enthält:
DMEM-Medium
   10 µg/l bFGF
   10 µmol/l 5-Azacytidin
   0,25 mg/l Amphotericin.

Ein Vergleich mit Kontrollansätzen ohne 5-Azazytidin zeigt, dass bei Stimulierung mit 5-Azazytidin deutlich mehr Stammzellen zu Kardiomyozyten differenzieren.

### BEISPIEL 4

### Differenzierung mit Cardiogenol

Die Zellen werden in einer Dichte von 1 x 10³ in Petrischalen ausgesät und direkt für 48 h mit einem Differenzierungsmedium kultiviert, welches enthält:
DMEM-Medium
   500 µl Cardiogenol-Lösung.

Für die Cardiogenol-Löung werden 5 mg Cardiogenol in 4,75 ml DMSO gelöst.

Auch hier entwickeln sich mehr Kardiomyozyten als in Kontrollen ohne Cardiogenol.

### BEISPIEL 5

### Differenzierung mit Insulin, Transferrin und PDGF

Die Zellen werden für 7 Tage in folgendem Differenzierungsmedium inkubiert:
DMEM-Medium
   820 µg/ml BSA
   5 µg/ml Transferrin
   5 µg/ml Insulin
   50 ng/ml PDGF

Auch hier entwickeln sich mehr Kardiomyozyten als in Kontrollen ohne Wachstumsfaktoren.

### BEISPIEL 6

### Differenzierung in Gegenwart von kokultivierten Kardiomyozyten

### 1. Variante:

Kardiomyozyten werden so auf eine Kulturflasche ausgesät, dass sie den Boden der Flasche komplett bewachsen. Dann werden Stammzellen (z.B. mit ß-Galaktosidase markiert) in einer Dichte von 1 x 10³ auf die Zellen gegeben und für 14 Tage kokultiviert. Anhand der markierten Stammzellen kann die Anzahl der in Kardiomyozyten differenzierten Zellen z.B. durch FACS-Analyse bestimmt werden.

### 2. Variante:

Kardiomyzyten werden in einem Zellkultur-Käfig für 14 Tage zu frisch ausgesäten pankreatischen Stammzellen gegeben. Die Kardiomyozyten werden verschiedene Substanzen abgeben, welche die Differenzierung der Stammzellen zu Kardiomyozyten fördern. Eine Fusion mit kokultivierten Zellen kann ausgeschlosen werden und die Zellen müssen vorher nicht markiert werden.

## Patentansprüche

1. Verfahren zur Bildung von Herzmuskelzellen (Kardiomyozyten), umfassend:
- eine Kultivierung und Differenzierung von Stammzellen, die aus differenziertem exokrinen Drüsengewebe aus dem Pankreas, einer Speicheldrüse oder Schweißdrüse eines Organismus gewonnen wurden, und
- Stimulation der Bildung der Herzmuskelzellen durch mindestens eine der folgenden Stimulationsbehandlungen:
- Co-Kultur mit Myokardzellen,
- Behandlung mit immobilisierten oder in einer Flüssigkeit gelösten molekularen Wachstums- oder Differenzierungsfaktoren.

2. Verfahren nach Anspruch 1, bei dem die Stimulation ferner die Aktivierung von mindestens einem Gen, das in die Differenzierung der Stammzellen zu den Herzmuskelzellen involviert ist, umfasst.

3. Verfahren zur Bildung von Herzmuskelzellen nach Anspruch 1 oder 2, umfassend
a) Kultivierung von Stammzellen, die aus differenziertem exokrinen Drüsengewebe aus dem Pankreas, einer Speicheldrüse oder Schweißdrüse eines Organismus gewonnen wurden,
b) weitere Kultivierung der Stammzellen in Gegenwart von Myokardgewebe,
c) Entfernung des Myokardgewebes und
d) weitere Kultivierung der Zellen und Überprüfung der Zellen hinsichtlich gebildeter Herzmuskelzellen,
e) Gewinnung der gebildeten Herzmuskelzellen.

4. Verfahren nach einem der vorhergehenden Ansprüche, bei dem eine Selektion der Herzmuskelzellen vorgesehen ist.

5. Verfahren nach Anspruch 4, bei dem die Selektion der Herzmuskelzellen ein Zellsortierverfahren umfasst.

6. Verfahren nach einem der vorhergehenden Ansprüche, bei dem Stammzellen aus Drüsengewebe verwendet werden, das acinäres Gewebe ist.

7. Verfahren nach einem der vorhergehenden Ansprüche, bei dem Stammzellen von einem Wirbeltier, vorzugsweise einem Säuger, verwendet werden.

8. Verfahren nach Anspruch 7, bei dem humane Stammzellen verwendet werden.

9. Verfahren nach einem der vorhergehenden Ansprüche, bei dem die Herzmuskelzellen für medizinische Anwendungen, insbesondere in der Regenerationsmedizin, verwendbar sind.

10. Stammzellenpatch (BTS) zur Wiederherstellung von verletztem oder geschädigtem Myokard, bei dem die in diesem Patch enthaltenen Stammzellen auf beiden Seiten (bidirektional) mit Zellen des angrenzenden Gewebes oder von den Zellen abgegebenen Substanzen in Kontakt kommen können, wodurch eine beidseitige Transformation/Differenzierung der Stammzellen, insbesondere zu Herzmuskelzellen (Kardiomyozyten), induziert oder stimuliert werden kann, umfassend
a) adulte Stammzellen, die aus exokrinem Drüsengewebe des Pankreas oder einer Speicheldrüse oder Schweißdrüse eines Organismus isoliert wurden,
b) eine poröse Matrix zur Aufnahme der Zellen,
c) eine breite Auflagefläche des BTS zur Auflage auf einer breiten Myokardwundfläche.

11. Stammzellenpatch nach Anspruch 10, **dadurch gekennzeichnet, dass** die Stammzellen humane Stammzellen sind.

12. Stammzellenpatch nach Anspruch 10 oder 11, **dadurch gekennzeichnet, dass** alle Bestandteile im Körper abbaubar sind.

13. Stammzellenpatch nach einem der Ansprüche 10-12, **dadurch gekennzeichnet, dass** die poröse Matrix eine Kollagenmatrix ist.

14. Stammzellenpatch nach einem der Ansprüche 10-13, **dadurch gekennzeichnet, dass** er ferner ganz oder teilweise zu Herzmuskelzellen ausdifferenzierte Zellen enthält.

15. Stammzellenpatch nach einem der Ansprüche 10-14, **dadurch gekennzeichnet, dass** er ferner andere im Herzen vorkommende differenzierte Zellen enthält.

16. Stammzellenpatch nach einem der Ansprüche 10-15, **dadurch gekennzeichnet, dass** er ferner Substanzen, welche die Differenzierung der Stammzellen zu Kardiomyozyten fördern, und/oder pharmazeutische Wirkstoffe, z.B. zur Unterdrückung einer Abstoßungsreaktion, enthält.

## Claims

1. A method for producing heart muscle cells (cardiomyocytes), comprising:
- cultivating and differentiating stem cells obtained from differentiated exocrine gland tissue from the pancreas, a salivary gland or sweat gland of an organism, and
- stimulation of the formation of the heart muscle cells by at least one of the following stimulation treatments:
- co-culture with myocardial cells,
- treatment with immobilised molecular growth or differentiation factors or with molecular growth or differentiation factors dissolved in a liquid.

2. The method for producing heart muscle cells according to claim 1, wherein the stimulation further comprises activation of at least one gene which is involved in the differentiation of the stem cells to heart muscle cells.

3. The method for producing heart muscle cells according to claim 1 or 2, comprising
a) cultivation of stem cells obtained from differentiated exocrine gland tissue from the pancreas, a salivary gland or sweat gland of an organism,
b) further cultivation of the stem cells in the presence of myocardial tissue,
c) removal of the myocardial tissue, and
d) further cultivation of the cells and testing the cells for the formation of heart muscle cells,
e) harvesting of the heart muscle cells formed.

4. The method according to one of the preceding claims, wherein selection of the heart muscle cells is provided.

5. The method according to claim 5, wherein the selection of the heart muscle cells includes a cell sorting procedure.

6. The method according to one of the preceding claims, wherein stem cells from glandular tissue which is acinar tissue are used.

7. The method according to one of the preceding claims, wherein stem cells from a vertebrate, preferably a mammal, are used.

8. The method according to claim 7, wherein human stem cells are used.

9. The method according to one of the preceding claims, wherein the heart muscle cells are usable for medical applications, in particular in regenerative medicine.

10. A stem cell patch (BTS) for regeneration of injured or damaged myocardium, wherein the stem cells contained within said patch can get into contact on both sides (bidirectional) with cells from the adjacent tissue or with substances produced by the cells, whereby a bidirectional transformation/- differentiation of the stem cells, in particular into heart muscle cells (cardiomyocytes), can be induced or stimulated, comprising
a) adult stem cells that have been isolated from exocrine gland tissue from the pancreas, a salivary gland or sweat gland of an organism,
b) a porous matrix for receiving the cells,
c) a broad supporting surface of the BTS for placement on a broad myocardial wound surface.

11. The stem cell patch according to claim 10, **characterized in that** the stem cells are human stem cells.

12. The stem cell patch according to claim 10 or 11, **characterized in that** all the constituents are degradable in the body.

13. The stem cell patch according to one of claims 10-12, **characterized in that** the porous matrix is a collagen matrix.

14. The stem cell patch according to one of claims 10-13, **characterized in that** it also contains cells entirely or partially differentiated out to heart muscle cells.

15. The stem cell patch according to one of claims 10-14, **characterized in that** it also contains other differentiated cells present in the heart.

16. The stem cell patch according to one of claims 10-15, **characterized in that** it also contains substances which promote the differentiation of the stem cells into cardiomyocytes and/or pharmaceutically active agents, for example, for suppressing a rejection reaction.

## Revendications

1. Procédé servant à produire des cellules myocardiques (cardiomyocytes), comprenant :
- une mise en culture et une différenciation de cellules souches, qui ont été extraites d'un tissu glandulaire exocrine différencié du pancréas, d'une glande salivaire ou d'une glande sudoripare d'un organisme, et
- une stimulation de la production des cellules myocardiques par au moins un des traitements de stimulation qui suit :
- la co-culture avec des cellules myocardiques,
- le traitement avec des facteurs de croissance ou de différenciation moléculaires immobilisés ou dissous dans un liquide.

2. Procédé selon la revendication 1, dans le cadre duquel la stimulation comprend en outre l'activation d'au moins un gène, qui est impliqué dans la différenciation des cellules souches par rapport aux cellules myocardiques.

3. Procédé servant à produire des cellules myocardiques selon la revendication 1 ou 2, comprenant
a) la mise en culture de cellules souches, qui ont été extraites d'un tissu glandulaire exocrine différencié du pancréas, d'une glande salivaire ou d'une glande sudoripare d'un organisme,
b) la poursuite de la mise en culture des cellules souches en présence d'un tissu myocardique,
c) l'élimination du tissu myocardique, et
d) la poursuite de la mise en culture des cellules et la vérification des cellules eu égard aux cellules myocardiques produites,
e) l'extraction des cellules myocardiques produites.

4. Procédé selon l'une quelconque des revendications précédentes, dans le cadre duquel une sélection des cellules myocardiques est prévue.

5. Procédé selon la revendication 4, dans le cadre duquel la sélection des cellules myocardiques comprend un procédé de tri de cellules.

6. Procédé selon l'une quelconque des revendications précédentes, dans le cadre duquel des cellules souches issues d'un tissu glandulaire sont utilisées, lequel tissu glandulaire est un tissu acinaire.

7. Procédé selon l'une quelconque des revendications précédentes, dans le cadre duquel des cellules souches provenant d'un animal vertébré, de préférence d'un mammifère, sont utilisées.

8. Procédé selon la revendication 7, dans le cadre duquel des cellules souches humaines sont utilisées.

9. Procédé selon l'une quelconque des revendications précédentes, dans le cadre duquel les cellules myocardiques peuvent être utilisées pour des applications médicales, en particulier dans la médecine de régénération.

10. Timbre de cellules souches (BTS) servant à restaurer le myocarde blessé ou endommagé, dans le cadre duquel les cellules souches contenues dans ledit timbre sur les deux côtés (bidirectionnel) peuvent venir en contact avec des cellules du tissu voisin ou avec des cellules de substances libérant des cellules, ce qui permet d'induire ou de stimuler une transformation/différenciation bilatérale des cellules souches, en particulier par rapport aux cellules myocardiques (cardiomyocytes), comprenant
a) des cellules souches adultes, qui ont été isolées d'un tissu glandulaire exocrine du pancréas ou d'une glande salivaire ou d'une glande sudoripare d'un organisme,
b) une matrice poreuse servant à recevoir les cellules,
c) une large surface de dépôt du BTS destinée à être déposée sur une large surface de lésion du myocarde.

11. Timbre de cellules souches selon la revendication 10, **caractérisé en ce que** les cellules souches sont des cellules souches humaines.

12. Timbre de cellules souches selon la revendication 10 ou 11, **caractérisé en ce que** tous les constituants sont dégradables dans le corps.

13. Timbre de cellules souches selon l'une quelconque des revendications 10 - 12, **caractérisé en ce que** la matrice poreuse est une matrice de collagène.

14. Timbre de cellules souches selon l'une quelconque des revendications 10 - 13, **caractérisé en ce qu'**il contient en outre, en totalité ou en partie par rapport aux cellules myocardiques, des cellules différenciées.

15. Timbre de cellules souches selon l'une quelconque des revendications 10 - 14, **caractérisé en ce qu'**il contient en outre d'autres cellules différenciées présentes dans le coeur.

16. Timbre de cellules souches selon l'une quelconque des revendications 10 - 15, **caractérisé en ce qu'**il contient en outre des substances, qui favorisent la différenciation des cellules souches par rapport aux cardiomyocytes, et/ou des principes actifs pharmaceutiques, par exemple servant à empêcher une réaction de rejet.
